# EUROPEAN PATENT APPLICATION

(11) **EP 2 905 330 A1**
(43) Date of publication of application: **12.08.2015**
(21) Application number: 13843761.1
(22) Date of filing: 27.09.2013
(51) Int. Cl.: C12M 3/04, C12N 1/00, C12N 5/071

(54) **CELL CULTURING MEMBER, AND CELL CULTURING METHOD**

(30) Priority: 05.10.2012 JP 2012222744
(71) Applicant: Nissha Printing Co., Ltd., Kyoto-shi, Kyoto 604-8551 (JP)
(72) Inventor: TABATA, Yasuhiko, Uji-shi Kyoto 611-0024 (JP); OBI, Naoko, Kyoto-shi Kyoto 604-8551 (JP); SUMIYOSHI, Chikako, Kyoto-shi Kyoto 604-8551 (JP); MURAKAMI, Hideki, Kyoto-shi Kyoto 604-8551 (JP)
(74) Representative: Kilian Kilian & Partner
(86) International application number: PCT/JP2013/076334
(87) International publication number: WO 2014/054538

(57) **Abstract**

[Problem] To provide a cell culture device capable of controlling the three-dimensional structure of cultured cell aggregates.

[Solution] Provided is a cell culture device 1 having a hydrogel layer 12 formed into a pattern on a substrate 11 and a layer of cell culture part 13 formed on the substrate to cover the hydrogel layer 12. The surface of the cell culture part functions as the plane 14 for seeding a first cell species. The hydrogel layer 12 is impregnated with a cell growth factor, and has a thickness which enables release of the cell growth factor from the hydrogel layer 12 into the cell culture part 13 throughout the cell culture period.

## Description

### Technical Field

The present invention relates to a cell culture device for *in vitro* culture of human and animal cells. The present invention also relates to a cell culture method to conduct an *in vitro* culture of human and animal cells.

### Background art

Cells in a living organism are arranged and disposed regularly and three-dimensionally. In the current cell culture technology, only a limited scope of technique is available for such arrangement and disposition of cultured cells.

There have been reported some techniques for two-dimensional cell arrangement. An example of the techniques provides a method in which cell-adhesive surface and non-cell adhesive surface are formed into patterns and used to control two-dimensional adhesion and subsequent growth of cells. In one of such known methods, a cell culture device is prepared by patterning fine lines of a temperature-responsive polymer on a substrate, and cells are cultured on the cell culture device (as disclosed in PTL 1). The cell culture device is able to control the structure of cultured cells two-dimensionally (along membrane surface), because the cell culture part of the cell culture device is patterned with fine lines of capillary width.

On the contrary, the technology and methodology for three-dimensional cell arrangement are almost unavailable. Although a technique of layering cell sheets to make three-dimensional cell arrangement has been reported (as disclosed in PTL 2), the technique fails to achieve intentional control of the inner structure of the multilayered cells. A prior art proposed a cell culture method in which a massive form of cells is made by growing cells three-dimensionally or sterically, though the method also fails to intentionally control three-dimensional cell arrangement.

In a three-dimensional or steric cell culture (culturing a massive form of cells), cells in the inner part of a massive form of cells are often fed with insufficient oxygen and nutrition, and the cells undergo functional decline or die. Contrary to the cultured cells, cells of three-dimensional aggregates in a living body do not face such problem, because blood vessels exist in the three-dimensional cell aggregates to supply sufficient oxygen and nutrition to cells inside the aggregates of a steric form.

As mentioned above, a vascular network is the key factor for cells to survive and function in three-dimensional cell aggregates. The vascular network is a product of three-dimensional arrangement of vascular cells. For the future advancement of cell biology, it is indispensable to develop a technique which controls cell arrangement in a form similar to tissue structure of a living body.

### Citation list

### Patent literature

PTL 1: Japanese Patent Publication Laid open 2010-98973
PTL 1: Japanese Patent Publication Laid open 2005-342112

### Summary of invention

### Technical problem

The problem to be solved by the present invention is to provide a cell culture device which can control the three-dimensional structure of cultured cells (aggregates of cells).

Another problem to be solved by the present invention is to provide a cell culture method which can control the three-dimensional structure of cultured cells (aggregates of cells). Yet another problem to be solved by the present invention is to provide a cell culture method which can control the three-dimensional structure of a cultured first cell species (aggregates of cells) and also co-culture a second cell species.

### Solution to problem

The cell culture device of the present invention controls the three-dimensional arrangement of cells by utilizing the property of cells responsive to the concentration gradient of a cell growth factor. At first, a hydrogel layer which gradually releases a cell growth factor is formed into a two-dimensional pattern on a substrate. Then a cell culture part (for example, a Matrigel layer) is placed on the patterned substrate, and cells are seeded on the cell culture part.

The cell growth factor is gradually released from the hydrogel layer into the cell culture part to make a three-dimensional concentration gradient. Then the cells migrate along the three-dimensional concentration gradient of the cell growth factor, and consequently the cells are arranged in the cell culture part according to the three-dimensional concentration gradient.

The method of the present invention enables three-dimensional cell arrangement in a cell culture part by means of the combination of a two-dimensionally patterned hydrogel and the property of the hydrogel gradually releasing a cell growth factor.

A cell culture device according to one embodiment of the present invention has a hydrogel layer being formed into a pattern on a substrate and a layer of cell culture part being formed on the substrate to cover the hydrogel layer, and the cell culture device is characterized in that
the surface of the cell culture part functions as the plane for seeding a first cell species,
the hydrogel layer is impregnated with a cell growth factor, and the cell growth factor is gradually released from the hydrogel layer into the cell culture part.

In a preferable embodiment of the cell culture device of the present invention, the hydrogel layer may have a thickness which enables continuous release of the cell growth factor from the hydrogel layer into the cell culture part for a period ranging from 2 days to 21 days, and the thickness may range from 1 µm to 30 mm.

In another preferable embodiment of the cell culture device of the present invention, the hydrogel layer may be composed of a thermally cross-linked gelatin, and the water content of a hydrogel constituting the hydrogel layer may range from 70 % to 99 %.

The cell culture method of the present invention includes the following steps of:
a) forming a hydrogel layer into a pattern on a substrate;
b) impregnating the hydrogel layer with a cell growth factor;
c) forming a layer of cell culture part on the substrate to cover the hydrogel layer and complete a cell culture device;
d) seeding a first cell species on the surface of the cell culture part; and
e) culturing the first cell species in the cell culture device under a culture condition.

In a preferable embodiment of the cell culture method of the present invention, the first cell species used in the seeding of the step d) may be starved before the seeding. The water content of a hydrogel constituting the hydrogel layer ranges from 70 % to 99 %, and may be controlled to adjust the period of gradual release of the cell growth factor from the hydrogel layer into the cell culture part.

In another preferable embodiment of the cell culture method of the present invention, the steps shown below follow the culturing of the first cell species of the step e):
f) seeding a second cell species on the surface of the cell culture part; and
g) culturing the second cell species in the cell culture device under a culture condition.

The present invention, preferable embodiments of the present invention and the elements contained therein can be combined as far as possible to work the invention.

### Advantageous effects of invention

The cell culture device of the present invention includes a hydrogel layer or a certain thickness in which a cell growth factor is impregnated. The hydrogel layer gradually releases the cell growth factor which makes the cultured first cell species migrate from the surface of the cell culture part toward the hydrogel layer and grow with the aid of the other matters specifying the invention. Such function of the hydrogel layer make it possible to control the two-dimensional structure of the cultured first cells (aggregate) along the pattern of the hydrogel layer (along the surface of the substrate), and also to control the structure of the cultured first cell species (aggregate) along the thickness of the cell culture part, in other words, the three-dimensional structure of the cultured first cell species.

The cell culture method of the present invention also make it possible to control the three-dimensional structure of cultured cells (aggregate) as well as the cell culture device mentioned above.

### Brief description of drawings

[Fig. 1] Fig. 1 is the cross-sectional view of a cell culture device.
[Fig. 2] Fig. 2 is the illustrative diagram of the cell culture device without a cell culture part. Fig.2 (a) is the cross-sectional view and Fig.2 (b) is the plan view.
[Fig. 3] Fig. 3 is the conceptual diagram of a cell culture method for a first cell species. Fig.3 (a), Fig.3 (b), and Fig.3 (c) are the conceptual diagrams respectively showing the state just after seeding a first cell species, an initial stage of the cell culture, and the final stage of the cell culture.
[Fig. 4] Fig. 4 is a graph showing the result from the determination of the example.

### Description of embodiment

The cell culture device and cell culture method of the present invention according to the embodiments of the present invention are further described referring to the figures. Some of the figures are pattern diagrams containing magnification of some elements for easy understanding of the present invention. Thus some of the dimensions or dimensional ratio between the elements may be different from that of an actual cell culture device and its parts. The dimensions, materials, forms, and relative positions of the members and parts described in the working examples of the present invention merely explain the present invention and do not restrict the scope of the present invention unless otherwise specified.

### Cell culture device

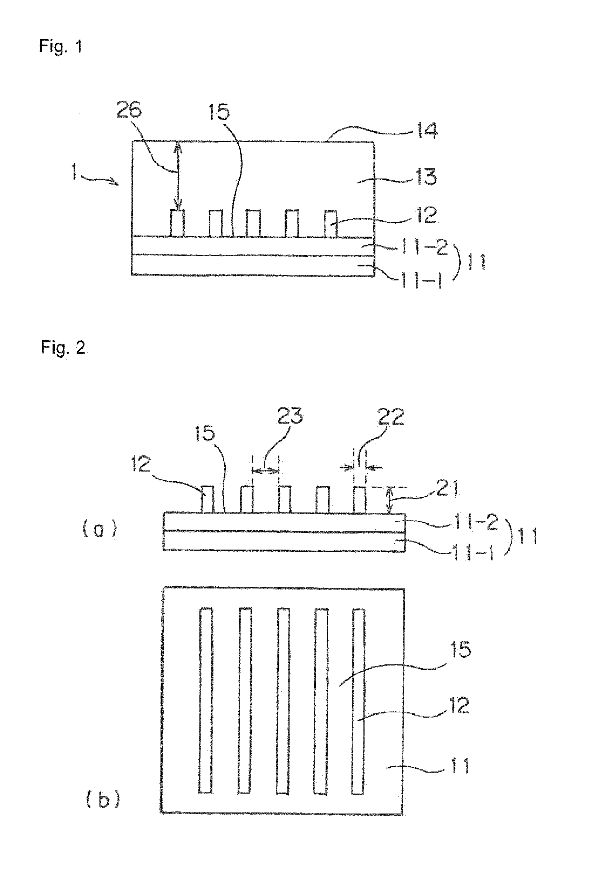

Fig. 1 is the cross-sectional view of a cell culture device 1, Fig. 2 is the illustrative diagram of the cell culture device 1 without a cell culture part, Fig.2 (a) is the cross-sectional view, and Fig.2 (b) is the plan view.

The cell culture device 1 is prepared by forming a hydrogel layer 12 on a substrate 11 and forming a cell culture part 13 covering the hydrogel layer 12. The hydrogel layer 12 is the carrier for gradual release of a cell growth factor. The hydrogel layer 12 also functions as a part to which growing first cell species adhere.

The substrate 11 is usually a flat plate, thin flat plate, or film. The hydrogel layer 12 is formed into a pattern on the substrate 11, and non-cell-adhesive part 15 is exposed at the area of the substrate on which the hydrogel layer 12 is not formed. The substrate 11 may consist of only a base 11-1, if the base 11-1 is made of a non-cell-adhesive material (for example, polydimethylsiloxane, hereinafter referred to as PDMS).

If the base 11-1 is made of a cell-adhesive material (for example, polyethylene terephthalate, hereinafter referred to as PET), a non-cell-adhesive layer 11-2 is formed by coating the whole surface of the base 11-1 with a non-cell-adhesive material. In the present invention, the non-cell-adhesive layer 11-2 is required or not required according to the material and surface characteristics of the base 11-1.

The material for the base 11-1 is selected from PDMS, PET, glass, silicone, metals (aluminum, stainless steel such as SUS 304, gold, silver, etc.), metal oxides (alumina, titanium oxide, ITO, and tin oxide), etc. The material for the non-cell-adhesive layer 11-2 is selected from agarose, polyethylene glycol, PDMS, etc.

The hydrogel layer 12 is formed into a pattern for the purpose of controlling the two-dimensional form (along the surface of the substrate 11) of the aggregates of cultured first cell species. As shown in Fig. 2(b), the hydrogel layer 12 in the embodiment is patterned into five straight lines arranged parallel. The line width (indicated by the arrow 22) of the patterned hydrogel layer is not specifically restricted, and preferably ranges from 10 µm to 500 µm. The space (indicated by the arrow 23) between the patterns of the hydrogel layer is not specifically restricted, and preferably ranges from 50 µm to 1000 µm.

The hydrogel layer 12 has a thickness which makes a volume of the hydrogel layer 12. Thus the hydrogel layer 12 can be impregnated with a cell growth factor which is stored in the layer and gradually released.

The hydrogel layer 12 is composed of a hydrogel in order to store and gradually release a cell growth factor.

The thickness (indicated by the arrow 21) of the hydrogel layer 12 is not specifically restricted. It is enough for the hydrogel layer 12 to have a thickness which allows the layer to contain an amount of a cell growth factor to be released sufficiently for a sufficient period for making seeded cell species migrate and grow toward the hydrogel layer 12.

The period of releasing a cell growth factor from the hydrogel layer can be adjusted by controlling the degree of cross-linking of the hydrogel constituting the layer. The water content of the hydrogel indicates the degree of cross-linking of the hydrogel. The preferable degree of cross-linking of the hydrogel is represented by the water content ranging usually from 70 % to 99 %, more preferably from 90 % to 99 %. A hydrogel which contains water within the above range will continue to release a cell growth factor in an amount sufficient for leading the direction of the growth of a first cell species throughout the culture period for the first cell species.

The desirable period of releasing a cell growth factor from the hydrogel layer preferably ranges from 2 days to 21 days. The thickness of the hydrogel layer preferably ranges from 1 µm to 3000 µm, more preferably from 20 µm to 70 µm. Such thickness enables the release of a cell growth factor in an amount sufficient for leading the direction of the growth of a first cell species throughout the culture period for the first cell species.

The preferable material for the hydrogel layer 12 includes gelatin, collagen, hyaluronic acid, fibronectin, laminin, fibrin, etc. Of those materials, gelatin is more preferable, because it can be thermally cross-linked by heating at relatively low temperature.

The hydrogel layer 12 may be formed optionally in several methods such as, 1) forming a thin hydrogel layer of a certain thickness on a glass plate, cutting the layer into a piece, and placing the piece on the substrate 11; 2) placing a gel material on the_substrate 11 with a dispenser: and 3) overprinting a hydrogel layer on the substrate 11 by screen printing.

The cell culture part 13 is formed into a layer covering the hydrogel layer 12, and has a surface parallel to the plane of the substrate. The hydrogel layer 12 is surrounded by the non-cell-adhesive part 15 on the surface of the substrate 11. The cell culture part 13 formed into a layer covers the hydrogel layer 12 and the non-cell-adhesive part 15.

The upper surface of the cell culture part 13 functions as the plane 14 for seeding a first cell species. The first cell species to be cultured is seeded on the plane 14 for seeding a first cell species of the cell culture device 1. The upper surface of the cell culture part 13 is one of the two surfaces of the layer of the cell culture part 13, and locates farther away from the substrate 11.

The first cell species grows in the cell culture part 13, where the cell growth factor is released from the hydrogel layer 12. A second cell species also grows in the cell culture part 13.

The thickness of the cell culture part 13, in other words, the distance (indicated by the arrow 26) between the surface of the hydrogel layer 12 and the plane 14 for seeding a first cell species should preferably range from 10 µm to 30 mm, more preferably from 100 µm to 5 mm. A thickness of the cell culture part 13 within the range effectively makes a first cell species grow toward the released cell growth factor, and consequently make the cell species form three-dimensional structure (extending along the thickness of the cell culture part).

A preferable material for the cell culture part 13 is Matrigel or collagen, and the collagen includes collagen gel and collage sponge.

### Cell culture method

The cell culture method for a first cell species is explained. A cell culture device is prepared prior to culturing the cell species.

The cell culture device is completed through the steps of 1) forming a hydrogel layer 12 into a pattern on a substrate 11, 2) impregnating the hydrogel layer 12 with a cell growth factor, and 3) forming the layer of cell culture part 13 covering the hydrogel layer 12.

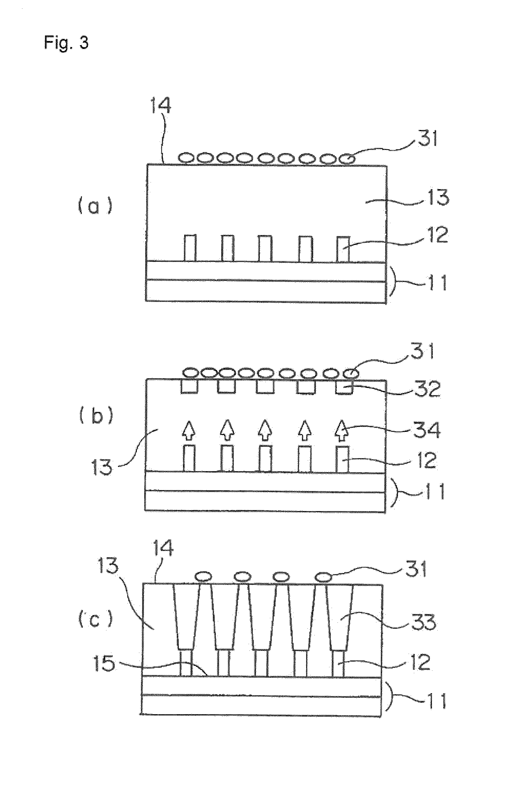

Fig. 3 is the conceptual diagram of a cell culture method for a first cell species. Fig. 3(a) shows the state just after seeding a first cell species, Fig.3 (b) shows an initial stage of the cell culture, and Fig.3 (c) shows the final stage of the cell culture.

A first cell species is seeded on the plane 14 for seeding a first cell species, which is the surface of the cell culture part, as shown in Fig. 3(a). The image of the first cell species seeded on the plane is indicated by the sign 31. Examples of the first cell species used in a preferable embodiment of the cell culture of the present invention include human umbilical vein endothelial cell (hereinafter referred to as HUVEC), fibroblast cell endothelial cell, epithelial cell, vascular endothelial cell, vascular smooth muscle cell, lymphatic endothelial cell, bile duct epithelial cell, and epidermal cell.

The cell growth factor used for the cell culture method and cell culture device of the present invention is not specifically restricted, and any of cell growth factors facilitating the growth and migration of the first cell species can be used. For example, VEGF (vascular endothelial growth factor) is employed for culturing HUVEC and lymphatic endothelial cells, and KGF (keratinocyte growth factor) is employed for culturing epidermal cells.

It is preferable to starve a first cell species 31 prior to seeding, because the starvation treatment accelerates the migration and growth of the first cell species toward the released cell growth factor.

The cell growth factor is released from the hydrogel layer 12 into the cell culture part 13 as shown in Fig. 3(b). The image of the released cell growth factor is indicated by the arrow 34. The seeded cells 31 migrate and grow toward the released cell growth factor. The image of the aggregates of the cultured cells at an initial stage of the cell culture is indicated by the sign 32 in Fig. 3(b).

The first cell species repeatedly migrate and grow toward the released cell growth factor during the cell culture. At the final stage of the cell culture, the aggregates of the cultured first cell species bridge the plane 14 for seeding a first cell species and the hydrogel layer 12. The image of the aggregates of the cultured cells at the final stage of the cell culture is indicated by the sign 33 in Fig. 3(c). Thus the three-dimensional structure (along the thickness of the cell culture part 13) of the aggregates of cultured cells is controlled.

The hydrogel layer 12 on the substrate 11 is surrounded by the non -cell- adhesive part 15 where the cultured cells do not extend. Such arrangement controls the two-dimensional structure (along the surface of the cell culture part 13) of the cultured cell aggregates.

HUVECs are cultured into aggregates of cultured cells 33 having tubular structure.

Then a preferable embodiment of the cell culture method of the present invention is described. In the cell culture device containing the aggregates of the cultured first cell species prepared as mentioned above, the second cell species is seeded. The second cell species is seeded on the cell culture part, and the cell culture device is kept under a cell culture condition to grow the second cell species. The cell culture condition varies depending on cell species to be cultured, and is selected from the conditions for known cell culture methods.

Thus two species of cells are co-cultured in an environment similar to that in a living body.

Examples of the second cell species include hepatic parenchymal cell, Ito cell, pancreatic parenchymal cell, cardiac muscle cell, nerve cell, mesenchymal cell, and tumor cell. Any combination of the first and second cell species may be employed.

### Example

### Example 1

### Preparation of a cell culture device (before forming a cell culture part)

A sheet of easily-adhesive PET film (Teijin® Tetoron® Film G274) placed on a hot plate (at 50 °C) was bar-coated with a 5-% aqueous solution of agarose (at 80°C), and dried in an oven at 40 °C for 30 minutes.

A glass plate was heated on a hot plate (at 50 °C) and coated with a 30-% aqueous solution of gelatin. After cooling, a piece of the gelatin was cut out and placed on the agarose-coated film described above. Then the film was cut into a gelatin-patterned substrate,

The gelatin-patterned substrate was vacuum-dried at 140°C for 48 hours to cross-link and sterilize the gelatin. The gelatin layer, i.e., the hydrogel layer, had a thickness of 50 µm, and was patterned into a 70-µm wide straight line.

HumanVEGF165 was employed as the cell growth factor. A piece of the cross-linked-gelatin-patterned substrate was immersed in an aqueous solution containing 40 ng/mL of human VEGF165 to impregnate the substrate with the solution. A control substrate was prepared by immersing a piece of the cross-linked-gelatin-patterned substrate in pure water to impregnate the substrate with the pure water.

### Preparation of the first cells

HUVECs were employed as the first cell species to be seeded. The HUVECs were cultured in a prescribed culture fluid, and then cultured in the mixture of Medium 199, 0.5 % of FCS (fetal calf serum), and 1 % of P/S (penicillin/streptomycin solution) for 1 day before seeding the cells. The cell culture in the mixture was conducted to starve the cells. The HUVECs were scraped after collagenase treatment just before the cell culture test, and fluorescently labeled with PKH26.

### Seeding and culturing

The substrate with the pattern of the human VEGF165-impregnated gelatin was placed in each well of a 24-well plate, and a Matrigel layer, i.e., a cell culture part, was formed in each of cell culture inserts for the 24-well plate. The concentration of the Matrigel in the layer was 10 mg/mL. Two types of cell culture devices were prepared respectively with a Matrigel layer formed of 20 µL of the Matrigel solution and a Matrigel layer formed of 50 µL of the Matrigel solution. The Matrigel layer of 20 µL of the Matrigel solution was about 0.7 mm thick while the layer of 50 µL of the Matrigel solution was about 1.7 mm thick. A control cell culture device was prepared by placing the substrate with the pattern of the pure-water-impregnated gelatin in each well of a 24-well plate, and by forming a Matrigel layer, i.e., a cell culture part, in each of cell culture inserts for the 24-well plate.

The starved HUVECs fluorescently labelled with PKH26 were seeded on the plane for seeding cell species, i.e., the surface of the Matrigel layer, and cultured in a CO₂-purged incubator for 21 hours.

### Determination of the cultured cells

After the cell culture, the number of HUVECs migrating to the bottom of the cell culture part (the Matrigel layer) was determined by measuring the fluorescence intensity at the bottom of the cell culture inserts with a plate reader.

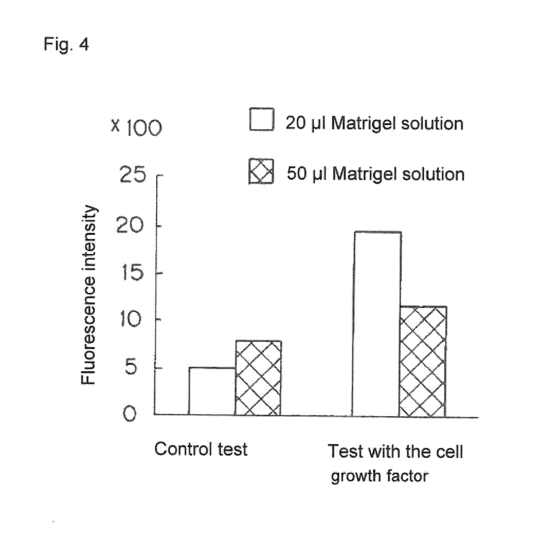

The result is shown in Fig. 4. The fluorescence intensity at the bottom of the cell culture inserts placed on the substrate patterned with human VEGF165-impregnated gelatin is stronger than the fluorescence intensity at the bottom of the cell culture inserts placed on the control substrate patterned with gelatin which was not impregnated with human VEGF165. The result implies that the HUVECs migrated through the cell culture part (the Matrigel layer) toward the human VGEF 165 which was released from the human VEGF165-impregnated gelatin patterned on the substrate.

### Reference Signs List

- 1: Cell culture device
- 11: Substrate
- 11-1: Base
- 11-2: Non-cell-adhesive layer
- 12: Hydrogel layer
- 13: Cell culture part
- 14: Plane for seeding a first cell species
- 15: Non-cell-adhesive part
- 31: First cell species seeded (image)
- 32: Aggregates of the cultured first cell species at an initial stage (image)
- 33: Aggregates of the cultured first cell species at the final stage (image)
- 34: Released cell growth factor (image)

## Claims

1. A cell culture device having
a hydrogel layer being formed into a pattern on a substrate, and
a layer of cell culture part being formed on the substrate to cover the hydrogel layer; the cell culture device being **characterized in that**
the surface of the cell culture part functions as the plane for seeding a first cell species,
the hydrogel layer is impregnated with a cell growth factor, and
the cell growth factor is gradually released from the hydrogel layer into the cell culture part.

2. A cell culture device according to Claim 1, wherein the hydrogel layer has a
thickness which enables continuous release of the cell growth factor from the hydrogel layer into the cell culture part for a period ranging from 2 days to 21 days.

3. A cell culture device according to Claim 1, wherein the hydrogel layer has a
thickness ranging from 1 µm to 30 mm.

4. A cell culture device according to any one of Claims 1 to 3, wherein the
hydrogel layer is composed of a thermally cross-linked gelatin.

5. A cell culture device according to any one of Claims 1 to 4, wherein the water
content of a hydrogel constituting the hydrogel layer ranges from 70 % to 99 %.

6. A cell culture method comprising the following steps off:
a) forming a hydrogel layer into a pattern on a substrate;
b) impregnating the hydrogel layer with a cell growth factor;
c) forming a layer of cell culture part on the substrate to cover the hydrogel layer and complete a cell culture devise:
d) seeding a first cell species on the surface of the cell culture part; and
e) culturing the first cell species in the cell culture device under a culture condition.

7. A cell culture method according to Claim 6, wherein the first cell species used
in the seeding of the step d) are starved before the seeding.

8. A cell culture method according to Claim 6 or 7, wherein the water content of a
hydrogel constituting the hydrogel layer ranges from 70 % to 99 %, and is controlled to adjust the period of gradual release of the cell growth factor from the hydrogel layer into the cell culture part.

9. A cell culture method according to Claim 6, wherein the steps shown below
follow the culturing of the first cell species of the step e):
f) seeding a second cell species on the surface of the cell culture part; and
g) culturing the second cell species in the cell culture device under a culture condition.
